# EUROPEAN PATENT APPLICATION

(11) **EP 1 988 079 A1**
(43) Date of publication of application: **05.11.2008**
(21) Application number: 07008412.4
(22) Date of filing: 25.04.2007
(51) Int. Cl.: C07D 215/18

(54) **Process for the preparation of optically active ethenylphenyl-alcohols**

(71) Applicant: Lonza AG, 4052 Basel (CH)
(72) Inventor: McGarrity, Johm, 3902 Brig-Glis (CH); Bappert, Erhard, 4056 Basel (CH)

(57) **Abstract**

Optically active ethenylphenyl-alcohols of formula 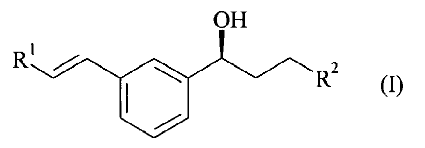 or its mirror image,
wherein R¹ is unsubstituted or substituted heteroaryl and R² is phenyl or substituted aryl, are prepared by asymmetric hydrogenation of the corresponding ketones applying hydrogen gas in the presence of specific platinum metal complex catalysts.

## Description

The present invention refers to a process for the preparation of optically active alcohols of formula 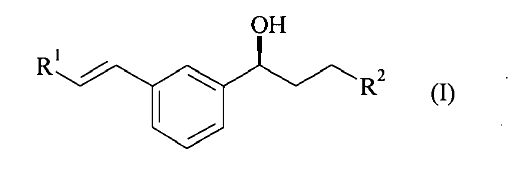 or its mirror image, wherein R¹ is unsubstituted or substituted heteroaryl and R² is phenyl or substituted aryl, by asymmetrically hydrogenating the corresponding ketones in the presence of specific platinum metal complex catalysts, particularly ruthenium.

Formula I comprises intermediates for the preparation of leukotriene antagonists being useful anti-asthmatic, anti-allergic, anti-inflammatory and cytoprotective therapeutic agents. Leukotriene antagonists are chiral compounds. One of their most prominent representatives is montelukast, the physiological active form of which is the (*R*) stereoisomer. The formation of the chiral center of formula I is a key step along the route to the final leukotriene antagonist as the hydrogenation of the corresponding ketones must be performed in a way that the alcohols obtained are optically enriched or pure. As these ketones additionally possess an olefinic bond, hydrogenation with standard hydrogenation catalysts followed by optical resolution is not the method of choice. The reason is that the olefinic bond is prone to be hydrogenated simultaneously with or even before the keto group. Therefore it is a big challenge to perform the hydrogenation both in a stereoselective and chemoselective manner.
It is known that the compounds of formula I can be obtained by reduction of the corresponding ketones with stoichiometric amounts of chiral reducing agents, particularly borane derivatives. For example EP 0 480 717 and US 2006/0223999 disclose use of oxazaborolidine complexes and EP 0 480 717 additionally describes application of *B*-chlorodiisopinocampheylborane ("DIP chloride"). If borane derivatives are the reducing agents, specialized and expensive facilities for the handling of these awkward and costly reagents are needed, especially for commercial production. Catalytic transfer hydrogenation is another approach for obtaining substances of formula I as disclosed in US 2006/008562. However, this kind of reaction results in moderate yields and low robustness of the reaction, while relatively large amounts of expensive catalyst are required.
Consequently, there is a high need for a catalytic asymmetric hydrogenation by which cheap hydrogen gas can be applied in standard, large-scale production units.

The object described above is achieved by the process of claim 1.

After numerous, unsuccessful experiments with different catalysts all providing the undesired ethenyl hydrogenation (see comparison examples), applicants have surprisingly found that it is possible to prepare optically active alcohols of formula 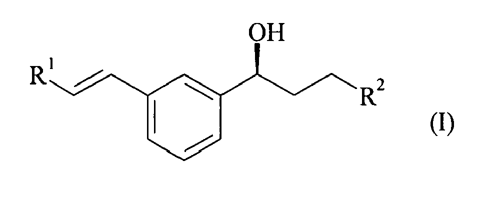 or its mirror image, wherein R¹ is unsubstituted or substituted heteroaryl and R² is phenyl or substituted aryl,
by asymmetric hydrogenation of a ketone of formula 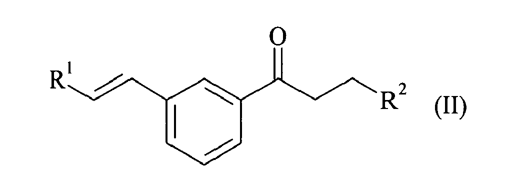 wherein R¹ and R² are as defined above,
with hydrogen gas in the presence of a platinum metal complex catalyst comprising a chiral phosphine ligand, wherein the platinum metal is selected from the group consisting of ruthenium, rhodium and iridium; and the chiral phosphine ligand is of formula 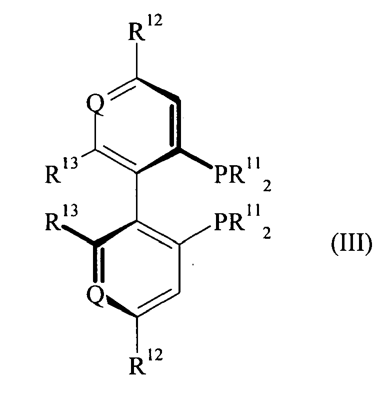 or its mirror image,
wherein
each R¹¹ is phenyl, 4-methylphenyl, 3,5-dimethylphenyl, furanyl or cyclohexyl;
each R¹² is hydrogen, C₁₋₄ alkyl or C₁₋₄ alkoxy;
and wherein
(a) each Q is nitrogen,
   and each R¹³ is C₁₋₄ alkyl or C₁₋₄ alkoxy; or
(b) each Q is =CR¹⁴-, R¹⁴ being selected from the group consisting of hydrogen, fluorine, chlorine, bromine, iodine and C₁₋₄ alkoxy,
   and each R¹³ is C₁₋₄ alkyl or C₁₋₄ alkoxy; or
(c) each Q is =CH-,
   and both R¹³ groups together form a moiety of formula
   -O-(CH₂)*ₙ*-O-, wherein n is an integer from 1 to 6; or
(d) each Q is =CR¹⁵-, and each R¹⁵ together with R¹³ bound to the same benzene ring and together with said benzene ring form a ring system selected from the group consisting of naphthalene, tetralin, 2,3-dihydro-benzo[1,4]dioxine, unsubstituted or substituted benzo[1,3]dioxole, and *N*-methyl-2,3-dihydro-benzo[1,4]oxazine.

Here and as follows, the term "platinum metal" means the group VIII transition metals ruthenium, rhodium, palladium, osmium, iridium and platinum.

Here and as follows, the term "C_{1-*n*} alkyl" is to be understood to mean any linear or branched alkyl group containing 1 to n carbon atoms. For example, the term "C₁₋₆ alkyl" comprises groups such as methyl, ethyl, propyl, isopropyl, butyl, isobutyl, *sec*-butyl, *tert*-butyl, pentyl, isopentyl (3-methylbutyl), neopentyl (2,2-dimethylpropyl), hexyl, isohexyl (4-methylpentyl) and the like.

Accordingly, the term "C_{1-*n*} alkoxy" means a group composed of a C_{1-*n*} alkyl group as defined above and an oxygen atom linked by a single covalent bond.

Here and as follows, the term "substituted benzo[1,3]dioxole" means a benzo[1,3]dioxole ring which is disubstituted at position 2 with two halogen, two C₁₋₄ alkyl or two C₁₋₄ alkoxy, respectively.

Here and as follows, the term "halogen" means fluorine, chlorine, bromine, iodine.

Examples of chiral phosphine ligands of formula III, in (*R*) or (*S*) configuration, are:
"P-Phos", wherein Q is nitrogen, R¹¹ is phenyl and R¹² and R¹³ are methoxy, i.e. 2,2',6,6'-tetramethyoxy-4,4'-bis(diphenylphosphino)-3,3'-bipyridine;
"Xyl-P-Phos", wherein Q is nitrogen, R¹¹ is 3,5-dimethylphenyl and R¹² and R¹³ are methoxy, i.e. 2,2',6,6'-tetramethoxy-4,4'-bis[di(3,5-dimethylphenyl)phosphino]-3,3'-bipyridine;
"Tol-P-Phos", wherein Q is nitrogen, R¹¹ is 4-methylphenyl and R¹² and R¹³ are methoxy, i.e. 2,2',6,6'-tetramethoxy-4,4'-bis[di(4-methylphenyl)phosphino]-3,3'-bipyridine;
"MeO-Biphep", wherein Q is =CR¹⁴-, R¹¹ is phenyl, R¹² is hydrogen, R¹³ is methoxy and R¹⁴ is hydrogen, i.e. 6,6'-dimethoxy-2,2'-bis(diphenylphosphino)-1,1'-biphenyl;
"3',5'-Me2-MeO-Biphep", wherein Q is =CR¹⁴-, R¹¹ is 3,5-dimethylphenyl, R¹² is hydrogen, R¹³ is methoxy and R¹⁴ is hydrogen, i.e. 6,6'-dimethoxy-2,2'-bis[di(3,5-dimethylphenyl)-phosphino]-1,1'-biphenyl;
"BIMOP", wherein Q is =CR¹⁴-, R¹¹ is phenyl, R¹² and R¹³ are methyl and R¹⁴ is methoxy, i.e. 5,5'-dimethoxy-4,4',6,6'-tetramethyl-2,2'-bis(diphenylphosphino)-1,1'-biphenyl;
   The compound, wherein Q is =CR¹⁴-, R¹¹ is 2-furanyl, R¹² is hydrogen, R¹³ is methoxy and R¹⁴ is hydrogen, i.e. 6,6'-dimethoxy-2,2'-bis[di(2-furanyl)phosphino]-1,1'-biphenyl;
"Cl-MeO-Biphep", wherein Q is =CR¹⁴-, R¹¹ is phenyl, R¹² is hydrogen, R¹³ is methoxy and R¹⁴ is chlorine, i.e. 6,6'-dimethoxy-5,5'-dichloro-2,2'-bis(diphenylphosphino)-1,1'-biphenyl;
"Bichep", wherein Q is =CR¹⁴-, R¹¹ is cyclohexyl, R¹² is hydrogen, R¹³ is methyl and R¹⁴ is hydrogen, i.e. 6,6'-dimethyl-2,2'-bis(dicyclohexylphosphino)-1,1'-biphenyl;
"Biphemp", wherein Q is =CR¹⁴-, R¹¹ is phenyl, R¹² is hydrogen, R¹³ is methyl and R¹⁴ is hydrogen, i.e. 6,6'-dimethyl-2,2'-bis(diphenylphosphino)-1,1'-biphenyl;
"C1-TunePhos", wherein Q is =CH-, R¹¹ is phenyl, R¹² is hydrogen, and both R¹³ together are -O-CH₂-O-, i.e. 6,6'-bis(diphenylphosphino)-2,2'-methylenedioxybiphenyl;
"C2-TunePhos", wherein Q is =CH-, R¹¹ is phenyl, R¹² is hydrogen, and both R¹³ together are -O-(CH₂)₂-O-, i.e. 1,12-bis(diphenylphosphino)-6,7-dihydro-dibenzo[*e,g]*[1,4]dioxocine;
"C3-TunePhos", wherein Q is =CH-, R¹¹ is phenyl, R¹² is hydrogen, and both R¹³ together are -O-(CH₂)₃-O-, i.e. 1,13-bis(diphenylphosphino)-7,8-dihydro-6*H*-dibenzo[*f,h*][1,5]dioxonine;
"C4-TunePhos", wherein Q is =CH-, R¹¹ is phenyl, R¹² is hydrogen, and both R¹³ together are -O-(CH₂)₄-O-, i.e. 1,14-bis(diphenylphosphino)-6,7,8,9-tetrahydrodibenzo[*g,i*][1,6]dioxecine;
"C5-TunePhos", wherein Q is =CH-, R¹¹ is phenyl, R¹² is hydrogen, and both R¹³ together are -O-(CH₂)₅-O-, i.e., 1,15-bis(diphenylphosphino)-8,14-dioxa-tricyclo[13.4.0.0^{2,7}]nonadeca-1(15),2(7),3,5,16,18-hexaene;
"C6-TunePhos", wherein Q is =CH-, R¹¹ is phenyl, R¹² is hydrogen, and both R¹³ together are -O-(CH₂)₆-O-, i.e. 1,16-bis(diphenylphosphino)-6,7,8,9,10,11-hexahydro-5,12-dioxa-di-benzo[*a*,*c*]cyclododecene;
"Binap", wherein Q is =CR¹⁵-, R¹¹ is phenyl, R¹² is hydrogen, and each R¹⁵ together with R¹³ bound to the same benzene ring and together with said benzene ring form a naphthalene ring system, i.e. 2,2'-bis(diphenylphosphino)-1,1'-binaphthalene;
"TolBinap", wherein Q is =CR¹⁵-, R¹¹ is 4-methylphenyl, R¹² is hydrogen, and each R¹⁵ together with R¹³ bound to the same benzene ring and together with said benzene ring form a naphthalene ring system, i.e. 2,2'-bis[di(4-methylphenyl)phosphino]-1,1'-binaphthalene;
"XylBinap", wherein Q is =CR¹⁵-, R¹¹ is 3,5-dimethylphenyl, R¹² is hydrogen, and each R¹⁵ together with R¹³ bound to the same benzene ring and together with said benzene ring form a naphthalene ring system, i.e. 2,2'-bis[di(3,5-dimethylphenyl)phosphino]-1,1'-binaphthalene;
"H₈-Binap", wherein Q is =CR¹⁵-, R¹¹ is phenyl, R¹² is hydrogen, and each R¹⁵ together with R¹³ bound to the same benzene ring and together with said benzene ring form a tetralin ring system, i.e. 6,6'-bis(diphenylphosphino)- 5,5'-bitetralin;
"Synphos", wherein Q is =CR¹⁵-, R¹¹ is phenyl, R¹² is hydrogen, and each R¹⁵ together with R¹³ bound to the same benzene ring and together with said benzene ring form a 2,3-dihydrobenzo[1,4]dioxine ring system, i.e. 2,2',3,3'-tetrahydro-6,6'-bis(diphenylphosphino)- 5,5'-bi(benzo[1,4]dioxine);
"Segphos", wherein Q is =CR¹⁵-, R¹¹ is phenyl, R¹² is hydrogen, and each R¹⁵ together with R¹³ bound to the same benzene ring and together with said benzene ring form a benzo[1,3]dioxole ring system, i.e. 5,5'-bis(diphenylphosphino)-4,4'-bi(benzo[1,3]dioxole);
"Difluorphos", wherein Q is =CR¹⁵-, R¹¹ is phenyl, R¹² is hydrogen, and each R¹⁵ together with R¹³ bound to the same benzene ring and together with said benzene ring form a 2,2-difluorobenzo[1,3]dioxole ring system, i.e. 2,2,2',2'-tetrafluoro-5,5'-bis(diphenylphosphino)-4,4'-bi-(benzo[1,3]dioxole);
   The compound, wherein Q is =CR¹⁵-, R¹¹ is phenyl, R¹² is hydrogen, and each R¹⁵ together with R¹³ bound to the same benzene ring and together with said benzene ring form a 2,2-dimethylbenzo[1,3]dioxole ring system, i.e. 2,2,2',2'-tetramethyl-5,5'-bis(diphenylphosphino)-4,4'-bi(benzo[1,3]dioxole);
"Solphos", wherein Q is =CR¹⁵ -, R¹¹ is phenyl, R¹² is hydrogen, and each R¹⁵ together with R¹³ bound to the same benzene ring and together with said benzene ring form a *N*-methyl-2,3-dihydro-benzo[1,4]oxazine, i.e. *N,N'*-dimethyl-2,2',3,3'-tetrahydro-7,7'-bis(diphenylphosphino) 8,8'-bi(benzo[1,4]oxazine) and
"XylSolphos", wherein Q is =CR¹⁵-, R¹¹ is 3,5-dimethylphenyl, R¹² is hydrogen, and each R¹⁵ together with R¹³ bound to the same benzene ring and together with said benzene ring form a *N-*methyl-2,3-dihydro-benzo[1,4]oxazine, i.e. *N,N*-dimethyl-2,2',3,3'-tetrahydro-7,7'-bis[di(3,5-dimethylphenyl)phosphino]-8,8'-bi(benzo[1,4]oxazine).

Particularly preferred are phosphines of formula III wherein Q is =CR¹⁵-, R¹² is hydrogen, and each R¹⁵ together with R¹³ bound to the same benzene ring and together with said benzene ring form a naphthalene ring system. The use of "Binap" has proved particularly advantageous.

In another preferred embodiment the herein disclosed platinum metal complex catalyst further comprises a chiral diamine ligand of formula 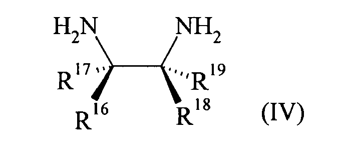 wherein R¹⁶ through R¹⁹ are each independently hydrogen, cycloalkyl, linear or branched C₁₋₆ alkyl, or phenyl optionally substituted with one or more C₁₋₄ alkyl or C₁₋₄ alkoxy groups, as these ligands showed a favourable effect on the chemoselectivity of the reaction.

The term "cycloalkyl" is to be understood to mean mono- or bicyclic saturated groups such as cyclopropyl, cyclobutyl, cyclopentyl, cyclohexyl, norbornyl, norcaryl, norpinanyl, and related groups, such as the above-mentioned groups being further substituted with lower alkyl substituents.

Examples of such chiral diamine ligands of formula IV are, particularly in (*R*) and (*S*) configuration for DAIPEN and in (*R,R*) and (*S,S*) configuration for DPEN:
"DAIPEN", wherein R¹⁶ is isopropyl; R¹⁷ is hydrogen; and R¹⁸ and R¹⁹ are 4-methoxyphenyl, i.e. 1,1-bis(4-methoxyphenyl)-3-methyl-1,2-butanediamine and
"DPEN", wherein R¹⁶ and R¹⁹ are phenyl; and R¹⁷ and R¹⁸ are hydrogen, i.e. 1,2-diphenylethylenediamine.

In a preferred embodiment the chiral diamine ligand of formula IV is "DAIPEN".

In a particular embodiment, R¹ is a heterocyclic group of formula 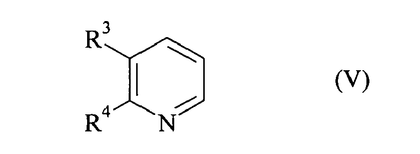 wherein R³ and R⁴ together form a moiety of formula -S-CR⁵=CR⁶-, with the proviso that the sulfur atom is directly bound to the carbon atom in position 3 of the pyridine moiety;
or alternatively R³ and R⁴ together form a moiety of formula -CR⁵=CR⁶-CR⁷=CR⁸-, with the proviso that the carbon atom attached to R⁵ is directly bound to the carbon atom in position 3 of the pyridine moiety;
and each of R⁵ through R⁸ is independently hydrogen or halogen.

Also particularly, R² is -C₆H₄R⁹, wherein R⁹ is selected from the group consisting of halogen, C₁₋₄ alkyl, branched and linear C₂₋₄ alkenyl, C₅₋₆ cycloalkyl, phenyl, C₁₋₄ alkoxy, C₁₋₄ alkylthio, carboxy, (C₁₋₄ alkoxy)carbonyl, (C₁₋₄ alkoxy)sulfonyl, -T-O-R¹⁰, wherein T is branched or linear C₁₋₈ alkanediyl and R¹⁰ is selected from the group consisting of hydrogen, methyl, substituted methyl, substituted ethyl, phenyl, substituted phenyl, substituted benzyl, pyridinylmethyl, substituted pyridinylmethyl, substituted silyl, C₁₋₆ acyl, substituted C₁₋₆ acyl, (C₁₋₄ alkoxy)carbonyl, substituted (C₁₋₄ alkoxy)carbonyl and aryloxycarbonyl; and R⁹ may be located at any position of the phenyl ring.

Here and as follows, the term "C_{2-*n*} alkenyl" is to be understood to mean a carbon chain containing at least one double bond located at any position of the carbon chain. For example, the term "C₂₋₄ alkenyl" comprises groups such as ethenyl, 1-methylethenyl, prop-1-enyl, prop-2-enyl, 2-methylprop-2-enyl and buta-1,3-dienyl.

Here and as follows, the term "C_{1-*n*} alkylthio" means a group composed of a C_{1-*n*} alkyl group as defined above and an sulfur atom linked by a single covalent bond.

Here and as follows, the term "(C_{1-*n*} alkoxy)carbonyl" means a carboxylic acid ester derived from a C_{1-*n*} alkanol which might be linear or branched. For example, the term "(C₁₋₄ alkoxy)-carbonyl" comprises groups such as methoxycarbonyl, ethoxycarbonyl, propoxycarbonyl, isopropoxycarbonyl, butoxycarbonyl, 2-methylpropoxy and *tert*-butoxycarbonyl.

Here and as follows, the term "(C_{1-*n*} alkoxy)sulfonyl" is analogous to the term "(C_{1-*n*} alkoxy)-carbonyl" as described above except for sulfonic acid ester instead of carboxylic acid ester.

Here and as follows, the term "substituted methyl" for R¹⁰ means a single carbon atom directly linked to the oxygen of the -T-O-R¹⁰ moiety while also linked at least to one heteroatom or carbon atom optionally forming a cyclic ring system thereby. Examples for "substituted methyl" are methoxymethyl, ethoxymethyl, methylthiomethyl, *tert*-butylthiomethyl, (phenyldimethylsilyl)methyoxymethyl, benzyloxymethyl, 4-methoxybenzyloxymethyl, (4-methoxyphenoxy)methyl, (2-methoxyphenoxy)methyl, *tert*-butoxymethyl, 4-pentenyloxymethyl, siloxymethyl, 2-methoxyethoxymethyl, 2,2,2-trichloroethoxymethyl, bis(2-chloroethoxy)methyl, 2-trimethylsilyl)ethoxymethyl, benzyl, diphenylmethyl, triphenylmethyl, tetrahydropyranyl, 3-bromotetrahydropyranyl, tetrahydrothiopyranyl, 1-methoxycyclohexyl, 4-methoxytetrahydropyranyl, 4-methoxytetrahydrothiopyranyl, *S*,*S*-dioxo-4-methoxytetrahydrothiopyranyl, 1-(2-chloro-4-methylphenyl)-4-methoxypiperidin-4-yl, 1,4-dioxan-2-yl, tetrahydrofuranyl, tetrahydrothiofuranyl and 2,3,3a,4,5,6,7,7a-octahydro-7,8,8-trimethyl-4,7-methanobenzofuran-2-yl.

Here and as follows, the term "substituted ethyl" for R¹⁰ means an ethyl group which in position 1 is directly linked to the oxygen of the -T-O-R¹⁰ moiety while said ethyl group is substituted in position 1 and/or position 2 by at least one substituent wherein the substituent is linked to the ethyl group by carbon-carbon, carbon-heteroatom, carbon-silicon and/or carbon-selenium bonding. Examples for "substituted ethyl" are 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, *tert*-butyl and allyl.

Here and as follows, the term "substituted phenyl" for R¹⁰ means a phenyl group directly linked to the oxygen of the -T-O-R¹⁰ moiety, wherein the phenyl is substituted at least with halogen, nitro, C₁₋₄ alkyl or C₁₋₄ alkoxy. Examples for "substituted phenyl" are 4-chlorophenyl, 4-methoxyphenyl and 2,4-dinitrophenyl.

Here and as follows, the term "substituted benzyl" for R¹⁰ means a carbon atom which is directly linked to the oxygen of the -T-O-R¹⁰ moiety and which is additionally linked to at least one substituted phenyl group. Examples for "substituted benzyl" are 4-methoxybenzyl, 3,4-dimethoxybenzyl, 2-nitrobenzyl, 4-nitrobenzyl, 4-halobenzyl, 2,6-dichlorobenzyl, 4-cyanobenzyl, 4-phenylbenzyl, 4,4'-dinitrobenzhydryl, 10,11-dihydro-5*H*-dibenzo[*a,d*]cycloheptene-5-yl, α-naphthyldiphenylmethyl, (4-methoxyphenyl)diphenylmethyl, di(4-methoxyphenyl)-phenylmethyl, tri(4-methoxyphenyl)methyl, [4-(4'-bromophenacyloxy)phenyl]diphenylmethyl, tris[4-(4,5-dichlorophthalimido)phenyl]methyl, tris[4-(4-oxopentanoyl)phenyl]methyl, tris(4-benzyloxyphenyl)methyl, [3-(imidazol-1-ylmethyl)phenyl]bis(4-methoxyphenyl)methyl, bis(4-methoxyphenyl)(1-pyrenyl)methyl, 9-phenylxanthene-9-yl and 9-phenyl-10-oxoanthracene-9-yl.

Here and as follows, the term "pyridinylmethyl" for R¹⁰ in the -T-O-R¹⁰ moiety comprises 2-pyridine-x-ylmethyl and 4-pyridine-x-ylmethyl.

Here and as follows, the term "substituted pyridinylmethyl" for R¹⁰ means a carbon atom which is directly linked to the oxygen of the -T-O-R¹⁰ moiety and which is additionally linked to at least one substituted pyridinyl group. An example for "substituted pyridinylmethyl" is *N*-oxy-3-methyl-2-pyridine-x-ylmethyl.

Here and as follows, the term "substituted silyl" for R¹⁰ means a silyl group which is directly linked to the oxygen of the -T-O-R¹⁰ moiety and which is substituted with substituents independently selected from the group consisting of linear or branched C₁₋₁₀ alkyl, C₁₋₃ alkoxy, benzyl and phenyl optionally substituted with one or more C₁₋₄ alkyl or C₁₋₄ alkoxy. Examples for "substituted silyl" are trimethylsilyl, triethylsilyl, triisopropylsilyl, dimethylisopropylsilyl, diethylisopropylsilyl, dimethylhexylsilyl, *tert*-butyldimethylsily, *tert*-butyldimethylsilyl, *tert-*butyldiphenylsilyl, tribenzylsilyl, tri-*p*-xylylsilyl, triphenylsilyl, diphenylmethylsilyl and *tert-*butyl-methoxy-phenylsilyl.

Here and as follows, the term "C₁₋₆ acyl" for R¹⁰ means an acyl group derived from a saturated or unsaturated carboxylic acid of 1 to 6 carbon atoms. Examples for "C₁₋₆ acyl" are formyl, acetyl, propanoyl and but-2-enoyl.

Here and as follows, the term "substituted C₁₋₆ acyl" for R¹⁰ means the acyl group as defined above which is substituted with at least one substituent independently selected from the group consisting of C₁₋₄ alkyl, halogen, oxo, C₁₋₄ alkoxy optionally substituted with phenyl, phenoxy, phenyl optionally substituted with one or more C₁₋₄ alkyl, phenyl or halogen, and 1-adamantyl. Examples for "substituted C₁₋₆ acyl" are 2-oxo-2-phenylacetyl, chloroacetyl, dichloroacetyl, trichloroacetyl, trifluoroacetyl, triphenylmethoxyacetyl, methoxyacetyl, phenoxyacetyl, 4-chlorophenylacetyl, 3-phenylpropanoyl, 4-oxopentanoyl, 2,2-dimethylpropanoyl, 1-adamantylformyl, 4-methoxybut-2-enoyl, benzoyl, 4-phenylbenzoyl and 2,4,6-trimethylbenzoyl.

Here and as follows, the term "substituted (C₁₋₄ alkoxy)carbonyl" for R¹⁰ means a (C₁₋₄ alkoxy)carbonyl group as defined above, wherein the C₁₋₄ alkanol from which it is derived is substituted with at least one substituent independently selected from the group consisting of halogen, C₁₋₄ alkyl substituted silyl, benzenesulfonyl, vinyl, phenyl optionally substituted with one or more C₁₋₄ alkyl, C₁₋₄ alkoxy, halogen, nitro and fluorenyl. Examples for substituted "(C₁₋₄ alkoxy)carbonyl" are 9-fluorenylmethoxycarbonyl, 2,2,2-trichloroethoxycarbonyl, 2-trimethylsilylethoxycarbonyl, 2-benzenesulfonylethoxycarbonyl, prop-2-enyloxycarbonyl, benzyloxycarbonyl, (4-methoxyphenyl)methoxycarbonyl, (3,4-dimethoxyphenyl)methoxycarbonyl, (2-nitrophenyl)methoxycarbonyl and (4-nitrophenyl)methoxycarbonyl.

Here and as follows, the term "aryloxycarbonyl" for R¹⁰ means a carboxylic acid ester derived from a C₆₋₁₈ phenol optionally substituted with one or more C₁₋₄ alkyl, C₁₋₄ alkoxy, halogen or nitro. An example for "aryloxycarbonyl" is 4-nitrophenoxycarbonyl.

In a preferred embodiment R¹ is of formula V, wherein R³ and R⁴ together form a moiety of formula -CR⁵=CR⁶-CR⁷=CR⁸-; and each of R⁵ through R⁸ is independently hydrogen or halogen; and R² is -C₆H₄R⁹, wherein R⁹ is (C₁₋₄ alkoxy)carbonyl or -T-O-R¹⁰, wherein T is branched or linear C₁₋₈ alkanediyl and R¹⁰ is hydrogen or substituted methyl; and R⁹ is located at position 2 of the phenyl ring.

In a more preferred embodiment R¹ is of formula V, wherein R³ and R⁴ together form a moiety of -CR⁵=CR⁶-CR⁷=CR⁸-; R⁵, R⁶ and R⁸ are hydrogen; and R⁷ is chlorine; and wherein R² is -C₆H₄R⁹, and R⁹ is -T-O-R¹⁰ wherein T is -C(CH₃)₂- and R¹⁰ is hydrogen or substituted methyl selected from the group consisting of tetrahydropyranyl, methoxymethyl and ethoxymethyl; and R⁹ is located at position 2 of the phenyl ring.

In a most preferred embodiment R¹ is of formula V, wherein R³ and R⁴ together form a moiety of formula -CR⁵=CR⁶-CR⁷=CR⁸-, R⁵, R⁶ and R⁸ are hydrogen and R⁷ is chlorine; and wherein R² is -C₆H₄R⁹, and R⁹ is methoxycarbonyl and located at position 2 of the phenyl ring, i.e. methyl 2-[3-[(*E*)-3-(2-(7-chloro-2-quinolinyl)ethenyl)phenyl]-3-oxopropyl]benzoate.

The catalyst can be obtained by dissolving a suitable salt of the platinum metal selected from the group consisting of of ruthenium, rhodium or iridium, wherein suitable counterions are for example chloride, bromide, iodide, tetrafluoroborate, hexafluoroarsenate, hexafluoroantimonate, hexafluorophosphate, perchlorate or trifluoromethanesulfonate in a polar solvent with a suitable amount of the phosphine ligand followed by isolation of the complex formed.

In addition, these suitable salts of ruthenium, rhodium or iridium preferably comprise at least one stabilizing ligand, such as an alkene, alkanediene or arene. In a preferred embodiment the stabilizing ligand is 2-methylallyl, 1,5-cyclooctadiene, norbornadiene, phenyl or *p*-cymene. The thus stabilized metal salts may also comprise at least one polar molecule as additional stabilizing ligand coming from the solvent or an added base. Examples of such polar molecules are acetonitrile, dimethylsulfoxide, dimethylformamide and triethylamine.

Alternatively, the catalyst can be prepared *in situ* from the phosphine ligands and the salt of the platinum metal as defined above. Preferably, the catalyst is prepared *in situ* from the phosphine ligands and salts of ruthenium, rhodium or iridium which are either stabilized as described above or which are suitable precursor complexes such as [RuCl₂(PPh₃)₃].

Optionally, the preparation of the catalyst can be performed in the presence of a chiral diamine ligand.

In a particularly preferred embodiment the salt is a ruthenium salt, the phosphine ligand is "Binap", and the chiral diamine is "DAIPEN". In a particularly preferred embodiment this ruthenium complex catalyst is [(*S*)-Binap RuCl₂ (*S*)-DAIPEN] and [(*R*)-Binap RuCl₂ (*R*)-DAIPEN].

A suitable preparation method of the "Binap" ligand is disclosed in D. Cai, J. F. Payack, D. R. Bender, D. L. Hughes, T. R. Verhoeven, P. J. Reider, Org. Synth. 1998, 76, 6-11. The isolated [Binap RuCl₂ DAIPEN] catalyst can be obtained by reacting [RuCl₂(C₆H₆)]₂, "Binap" and "DAIPEN" in dimethylformamide followed by re-crystallization from dichloromethane and diethyl ether (1:10).

The catalyst may be added to the reaction mixture as such or dissolved in a suitable solvent, or alternatively the catalyst may be prepared *in situ.*

The catalyst may also be polymer-bound by linkage of a suitable group of the phosphine ligand to a resin. Polymer-bound catalysts of this kind are particularly advantageous for simple purification of the product.

Bases applicable in the present invention include inorganic and organic bases. The bases may be expressed by the general formula MY, wherein M is an alkali metal or one equivalent of an alkaline earth metal, and Y is a hydroxy group, alkoxy group, carboxylate, hydrogencarbonate or one equivalent of carbonate. More specifically, applicable bases include NaOH, KOH, CsOH, LiOCH₃, NaOCH₃, NaOCH(CH₃)₂, KOCH₃, KOCH(CH₃)₂, KOC(CH₃)₃, NaOCOCH₃, K₂CO₃, Na₂CO₃, Cs₂CO₃, CaCO₃ and BaCO₃. Alternatively, the base may be an amine like *tert-*butylamine, dimethylamine, diethylamine, trimethylamine, triethylamine or triethylenediamine. Most preferably K₂CO₃, Cs₂CO₃ or NaOH is used as base.

As solvent, any inert liquid solvent which can dissolve the reactants and catalyst components may be used. Applicable solvents include aromatic hydrocarbons such as toluene and xylene; halogenated aromatic hydrocarbons such as chlorobenzene and trifluorotoluene; aliphatic hydrocarbons such as pentane and hexane; halogenated hydrocarbons such as dichloromethane and dichloroethene; ethers such as diethyl ether, tetrahydrofuran, 2-methyltetrahydrofuran; alcohols such as methanol, ethanol, 2-propanol, butanol and benzyl alcohol; halogenated alcohols such as 2,2,2-trifluoroethanol; carboxylic esters and lactones such as ethyl acetate, methyl acetate and valerolactone; and organic solvents containing heteroatoms such as acetonitrile, dimethylformamide and dimethyl sulfoxide. The solvents can be used alone; as mixture or two-phase system with water; or in mixtures of at least two solvents optionally in combination with water. Preferred solvents are mixtures of halogenated aromatic hydrocarbons and alcohols.

Optionally, the reaction mixture may contain a Lewis acid such as scandium(III) triflate, bismuth(III) triflate, yttrium(III) triflate, copper(I) chloride, copper(II) chloride, magnesium chloride, aluminium chloride, iron(III) chloride, cerium(III) chloride, lanthanum(III) chloride, neodymium(III) chloride and samarium(III) chloride. If a Lewis acid appears as hydrate, this hydrate compound may be applied as well. Addition of Lewis acids may enhance both the enantioselectivity of the reaction and the stability of the catalyst.

Optionally, the reaction can be carried out in the presence of a phase transfer catalyst such as an ammonium halide. Examples of such ammonium halides are tetraethylammonium bromide, triethylbenzylammonium chloride (TEBA), tetrabutylammonium chloride, tetrabutylammonium bromide and tetrabutylammonium iodide. TEBA has been found to be particularly useful. Addition of phase transfer catalysts may have a positive effect on the separation of the product formed.

The amount of the ketone of formula II (substrate) varies with the reactor volume and can be at a molar ratio relative to the catalyst (S/C) from 100:1 1 to 100,000:1, or more preferably from 500:1 to 20,000:1.

The hydrogenation process according to the invention may be carried out at atmospheric pressure or superatmospheric pressure. Typical pressures are from 1 to 100 bar. Advantageously, 1 to 70 bar, in particular 5 to 40 bar are used. The chemoselectivity appears to be generally better with lower pressures.

The hydrogenation reactions may be carried out at low or elevated temperatures. An examplary temperature range is from -20 °C to 120 °C. Preferred is a temperature between 0 °C and 100 °C, and most preferred is a range from 10 °C to 40 °C.

The reaction time depends on different factors like the catalyst loading, the temperature and the hydrogen pressure. Therefore, the reaction may be completed in a period of time within a range from a few minutes to several hours or even days.

### Examples

The following examples further illustrate this invention but are not intended to limit it in any way.

The structure and stereochemistry of the comparison ligand 1 is as follows:

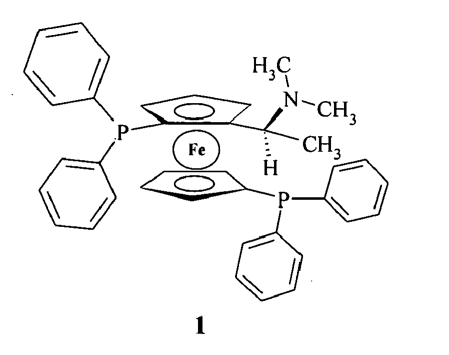

### Example 1: Synthesis of 1-[(E)-3-(2-(7-chloro-2-quinolinyl)ethenyl)phenyl]-2-propen-1-ol

A suspension of (*E*)-3-(2-(7-chloro-2-quinolinyl)ethenyl)benzaldehyde (50 g, 0.17 mol, commercially available from Unibest Industrial Ltd., Ningbo, China) in 400 mL toluene was degassed at 0 °C. A 1.6 M solution of vinylmagnesium chloride in THF (115 mL, 0.18 mol) was added dropwise over 30 minutes while keeping the internal temperature at <10 °C. After stirring for 1 hour at 0-5 °C, the reaction mixture was quenched by slow addition of 400 mL aqueous ammonium acetate solution (10 %). The two-phase mixture thus obtained was stirred for 1 hour to ensure the solvolysis of the magnesium salts.
The separated organic layer was washed two times with 500 mL water and concentrated in vacuum to a volume of 75 mL. Then, again 75 mL acetonitrile were added and the mixture was concentrated in vacuum to 75 mL. After repetition of the last procedure, the resulting slurry was directly used in the next step.

### Example 2: Synthesis of methyl 2-[3-[(E)-3-(2-(7-chloro-2-quinolinyl)ethenyl)phenyl]-3-oxopropyl]benzoate

The crude slurry of example 1 in acetonitrile was treated with methyl 2-iodobenzoate (44.6 g, 0.17 mol), triethylamine (35.6 mL, 0.254 mol) and palladium acetate (0.36 g, 1.7 mmol). The mixture was degassed and heated at reflux under nitrogen for 6 hours. Then, the hot solution was filtered through cellulose (Solka Floc^{®}) to remove any precipitated palladium. When the filtrate cooled to ambient temperature, the desired title product crystallized from solution. After one hour at ambient temperature, the suspension was filtered. The filter cake was washed consecutively with 130 mL acetonitrile, 100 mL acetonitrile/water (1:1), 100 mL water and finally 150 mL acetonitrile. After drying, the title product was obtained as a pale-yellow solid (53 g, 69% referring to the benzaldehyde of Example 1).
¹H NMR complies with EP 0 480 717 B (example 16, step 3).

### Examples 3 to 9 and comparison examples C1 to C3: Asymmetric hydrogenation of methyl 2-[3-[(E)-3-(2-(7-chloro-2-quinolinyl)ethenyl)phenyl]-3-oxopropyl]benzoate

The examples 3 to 6 and C 1 to C3 were performed by high throughput catalysis screening (HTS) using a HTS automated screening tool from Symyx Inc. and a customized Symyx Workflow. All reactions were performed in 1.2 mL vials on a 96-well plate placed in a high-pressure reactor (HIP). The whole HTS was performed in a glove box.

### Procedure for examples 3 to 6 - exemplarily shown on example 3:

A stock solution of [(*S*)-Binap RuCl₂ (*S*)-DAIPEN] in dichloroethene (0.0017 mmol in 0.08 mL) was prepared and filled into a reaction vial. The solvent was completely removed under reduced pressure. A stock solution of the substrate methyl 2-[3-[(*E*)-3-(2-(7-chloro-2-quinolinyl)-ethenyl)phenyl]-3-oxopropyl]benzoate in tetrahydrofuran (0.042 mmol in 0.09 mL) was added. The solvent was completely removed under reduced pressure a second time. Stock solutions of potassium carbonate in water (0.046 mmol in 0.05 mL) and of triethylbenzylammonium chloride in 2-propanol (0.004 mmol in 0.06 mL) were added. Finally, the vial was filled with toluene until a total volume of 0.5 mL was reached. The reaction mixture was then purged four times with hydrogen, pressurised to 5 bar and run for 18 hours at room temperature.

### General procedure for examples 7 to 9:

For example 8, the reaction mixture and hydrogenation conditions are identical to example 10, except for the reaction time. For examples 7 and 9, the reaction mixture was prepared analogous to example 10, but hydrogenation was performed in a 50 mL stainless steel autoclave and the product was isolated only by extraction.

### Procedure for the comparison examples C 1 to C3 - exemplarily shown on example C1:

A solution of the ligand 1 in dichloroethene (0.0020 mmol in 0.12 mL) was filled into a reaction vial. A stock solution of [RuI₂(*p*-cymene)]₂ in dichloroethene (0.0008 mmol in 0.04 mL) was added, followed by toluene until a total volume of 0.40 mL was reached. The vial was closed, and the mixture was heated at 80 °C for one hour. Then, the solvent was completely removed under reduced pressure and a stock solution of the substrate methyl 2-[3-[(*E*)-3-(2-(7-chloro-2-quinolinyl)ethenyl)phenyl]-3-oxopropyl]benzoate in tetrahydrofuran (0.042 mmol in 0.09 mL) was added. A stock solution of lithium methanolate in methanol (0.033 mmol in 0.10 mL) was also added. Finally, the vial was filled with 2-propanol until a total volume of 0.50 mL was reached. The reaction mixture was then purged four times with hydrogen, pressurized to 5 bar and run for 16 hours at room temperature.

Details of the examples 3 to 9 and C1 to C3 with regard to the catalyst used, reaction conditions and the results achieved are compiled in tables 1 and 2. Conversion and the product methyl 2-[3-[(*E*)-3-(2-(7-chloro-2-quinolinyl)ethenyl)phenyl]-3-hydroxypropyl]benzoate were calculated from uncorrected supercritical fluid chromatography (SFC) integrals. The enantiomeric excess (ee) was also measured by SFC (Chiralpak AD-H, 40% 2-propanol).

**Table 1: Examples 3 to 9 (room temperature, 5 bar of hydrogen)**

| No | Cat | S/C | S | Solvent | Base/ | Time | Conv | Product | ee |
|---|---|---|---|---|---|---|---|---|---|
| | | | [mmol] | mixture | Additive * | [h] | [%] | [%] | [%] |
| 3 | *(S)* | 25 | 0.042 | IPA/toluene/H₂O | K₂CO₃/ | 18 | 100 | 98.3 | 95.4 |
| | | | | (1:6:1; 0.5 mL) | TEBA | | | | (R) |
| 4 | *(S)* | 25 | 0.042 | Clbenz/THF/H₂O | K₂CO₃/ | 18 | 100 | 95.5 | 94.9 |
| | | | | (15:1:2; 0.5 mL) | Y(OTF)₃ | | | | (R) |
| 5 | *(S)* | 25 | 0.042 | IPA, triFtoluene | Cs₂CO₃/ | 6 | 100 | 94.3 | 96.4 |
| | | | | (1:4; 0.5 mL) | --- | | | | (R) |
| 6 | *(S)* | 100 | 0.083 | Clbenz/IPA/H₂O | NaOH/ | 6 | 99.4 | 97.6 | 95.7 |
| | | | | (5:1:1.5; 0.5 mL) | TEBA | | | | (R) |
| 7 | *(R)* | 100 | 3.5 | Clbenz/IPA | NaOH/ | 6 | 100 | > 99 | 96.9 |
| | | | | (5:1; 20 mL) | TEBA | | | | (*S*) |
| 8 | *(R)* | 482 | 31.8 | Clbenz/IPA | NaOH/ | 6 | > 95 | 99 | 96.7 |
| | | | | (5:1; 20 mL) | TEBA | | | | (*S*) |
| 9 | *(R)* | 1000 | 3.5 | Clbenz/IPA | NaOH | 30 | 99 | 99 | 96.6 |
| | | | | (5:1; 20 mL) | TEBA | | | | (*S*) |

| | | | | | | | | | |
|---|---|---|---|---|---|---|---|---|---|
| * = 1.1 equivalents base and 0.05-0.1 equivalents additive; cat = catalyst; *(S)* = [(*S)*-Binap RuCl₂ *(S)*-DAIPEN]; *(R)* = [(*R*)-Binap RuCl₂ *(R)*-DAIPEN]; (*S*)-Binap = (*S*)-(-)-2,2'-bis-(diphenylphosphino)-1,1'-binaphthalene; (*S*)-DAIPEN = (2*S*)-(-)-1,1-bis(4-methoxyphenyl)-3-methyl-1,2-butanediamine; IPA = 2-propanol; THF = tetrahydrofuran; Cl-benzene = chlorobenzene; triFtoluene = trifluorotoluene; Y(OTF)₃ = yttrium(III) triflate, TEBA = triethylbenzylammonium chloride. | | | | | | | | | |

**Table 2: Comparison examples C1 to C3 (IPA/THF/MeOH 3:1:1; 0.5 mL; room temperature; 5 bar of hydrogen)**

| No | Pre | Lig | S/C | S | Base/ | Time | Conv | Product | By-Product |
|---|---|---|---|---|---|---|---|---|---|
| | | | | [mmol] | Additive * | [h] | [%] | [%] | (C=C hydrogenation) |
| | | | | | | | | | [%] |
| C1 | **a** | **1** | 25 | 0.042 | LiOMe/ | 16 | 82.6 | 0 | 54.1 |
| | | | | | --- | | | | |
| C2 | **b** | **1** | 25 | 0.042 | DABCO/ | 16 | 84.3 | 0 | 75.0 |
| | | | | | --- | | | | |
| C3 | **a** | **2** | 25 | 0.042 | LiOMe/ | 16 | 90.3 | 3.9 | 44.9 |
| | | | | | --- | | | | |

| | | | | | | | | | |
|---|---|---|---|---|---|---|---|---|---|
| Pre = precursor catalyst; Lig = ligand; * = 1.1 equivalents base; **a** = [RuI₂(*p*-cymene)]₂; **b** = [Ir(1,5-cyclooctadiene)₂]BARF with BARF = tetrakis[3,5-bis(trifluoromethyl)phenyl]borate; **1** = for structure see above; **2** = (R)-Xyl-P-Phos; IPA = 2-propanol; THF = tetrahydrofuran; MeOH = methanol; LiOMe = lithium methanolate; DABCO = triethylenediamine. | | | | | | | | | |

### Example 10: Use of [(R)-Binap RuCl₂ (R)-DAIPEN] as catalyst

14.50 g (31.80 mmol) of methyl 2-[3-[(*E*)-3-(2-(7-chloro-2-quinolinyl)ethenyl)phenyl]-3-oxopropyl]benzoate and 375 mg (1.64 mmol) triethylbenzylammonium chloride were placed in a 300 mL stainless steel autoclave equipped with a hollow shaft stirrer and a sampling tube under argon. 25 mL degassed 2-propanol and 36.2 mL (36.2 mmol) degassed, aqueous sodium hydroxide solution [1 M] was successively added. 72.95 mg (0.066 mmol, S/C = 482:1) of [(*R*)-Binap RuCl₂ (*R*)-DAIPEN] was set under argon and dissolved in 120 mL of dry and degassed chlorobenzene. The catalyst solution was transferred into the autoclave under argon. Then, the autoclave was purged three times each with argon and hydrogen before the hydrogen pressure was set on a value of 5 bar at room temperature. The progress of the reaction was followed by taking samples after 3 and 6 hours. In addition, a hydrogen uptake curve was measured. After 8 hours no further hydrogen consumption was observed so that the pressure was released and the resulting heterogeneous mixture was divided into two equal portions. The two portions were subjected to two different work-up procedures resulting in a total of 13.37 g (92%) of the desired product methyl 2-[3-[(*E*)-3-(2-(7-chloro-2-quinolinyl)ethenyl)phenyl]-3(*S*)-3-hydroxypropyl]benzoate:

### a) Non-aqueous work-up (filtration)

The first portion was directly transferred into a glass filter and filtered. The precipitate thus obtained was washed with 50 mL water, 50 mL toluene and 30 mL ethyl acetate/hexane (1:1). After drying, 3.96 g (27%) of methyl 2-[3-[(*E*)-3-(2-(7-chloro-2-quinolinyl)ethenyl)phenyl]-3(*S*)-3-hydroxypropyl]benzoate was obtained as tan, crystalline powder.
The mother liquor and the washing solutions were applied to an aqueous work-up as outlined above. Thus, an additional amount of 2.11 g (15%) of methyl 2-[3-[(*E*)-3-(2-(7-chloro-2-quinolinyl)ethenyl)phenyl]-3(*S*)-3-hydroxypropyl]benzoate was obtained.

### b) Aqueous work-up (extraction)

The second portion was treated with 600 mL ethyl acetate and 200 mL water. The layers were separated and the aqueous layer was extracted twice with 300 mL ethyl acetate. The combined organic layers were dried over sodium sulfate. Finally, the solvent was removed under reduced pressure yielding 7.30 g (50%) of methyl 2-[3-[(*E*)-3-(2-(7-chloro-2-quinolinyl)ethenyl)-phenyl]-3(*S*)-3-hydroxypropyl]benzoate as a resinous orange solid.

Purity: 98% by SFC (Chiralpak AD-H, 40% 2-propanol). Enantiomeric purity: 96.4% ee (*S*) by SFC (same conditions).

## Claims

1. A process for the preparation of an optically active alcohol of formula 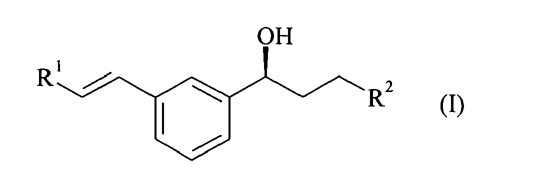 or its mirror image, wherein R¹ is unsubstituted or substituted heteroaryl and R² is phenyl or substituted aryl,
by asymmetric hydrogenation of a ketone of formula 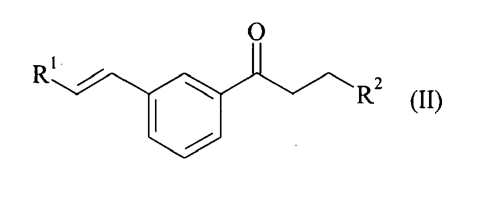 wherein R¹ and R² are as defined above,
**characterized in that** the asymmetric hydrogenation is conducted with hydrogen gas in the presence of a platinum metal complex catalyst comprising a chiral phosphine ligand,
wherein
the platinum metal is selected from the group consisting of ruthenium, rhodium and iridium; and the chiral phosphine ligand is of formula 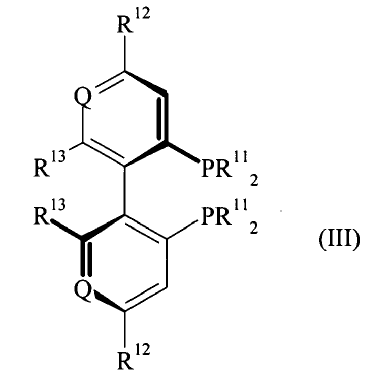 or its mirror image,
wherein
each R¹¹ is phenyl, 4-methylphenyl, 3,5-dimethylphenyl, furanyl or cyclohexyl;
each R¹² is hydrogen, C₁₋₄ alkyl or C₁₋₄ alkoxy;
and wherein
(a) each Q is nitrogen,
and each R¹³ is C₁₋₄ alkyl or C₁₋₄ alkoxy; or
(b) each Q is =CR¹⁴-, R¹⁴ being selected from the group consisting of hydrogen, fluorine, chlorine, bromine, iodine and C₁₋₄ alkoxy;
and each R¹³ is C₁₋₄ alkyl or C₁₋₄ alkoxy; or
(c) each Q is =CH-,
and both R¹³ groups together form a moiety of formula -O-(CH₂)*ₙ*-O-, wherein *n* is an integer from 1 to 6; or
(d) each Q is =CR¹⁵-, and each R¹⁵ together with R¹³ bound to the same benzene ring and together with said benzene ring form a ring system selected from the group consisting of naphthalene, tetralin, 2,3-dihydro-benzo[1,4]dioxine, unsubstituted or 2,2-dihalogen substituted benzo[1,3]dioxole, and *N*-methyl-2,3-dihydro-benzo[1,4]oxazine.

2. The process of claim 1, wherein the platinum metal complex catalyst further comprises a chiral diamine ligand.

3. The process of claim 2, wherein the chiral diamine ligand is of formula 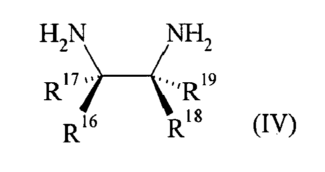 wherein R¹⁶ through R¹⁹ is each independently hydrogen, cycloalkyl, linear or branched C₁₋₆ alkyl, or phenyl optionally substituted with one or more C₁₋₄ alkyl or C₁₋₄ alkoxy groups.

4. The process of claim 3, wherein R¹⁶ is isopropyl; R¹⁷ is hydrogen; and R¹⁸ and R¹⁹ are 4-methoxyphenyl.

5. The process of any of claims 1 to 4, wherein R¹ is a heterocyclic group of formula 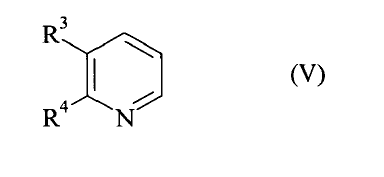 wherein R³ and R⁴ together form a moiety of formula -S-CR⁵=CR⁶-, with the proviso that the sulfur atom is directly bound to the carbon atom in position 3 of the pyridine moiety;
or alternatively R³ and R⁴ together form a moiety of formula -CR⁵=CR⁶-CR⁷=CR⁸-, with the proviso that the carbon atom attached to R⁵ is directly bound to the carbon atom in position 3 of the pyridine moiety;
and each of R⁵ through R⁸ is independently hydrogen or halogen.

6. The process of any of claims 1 to 5, wherein R² is -C₆H₄R⁹, wherein R⁹ is selected from the group consisting of halogen, C₁₋₄ alkyl, branched or linear C₂₋₄ alkenyl, C₅₋₆ cycloalkyl, phenyl, C₁₋₄ alkoxy, C₁₋₄ alkylthio, carboxy, (C₁₋₄ alkoxy)carbonyl , (C₁₋₄ alkoxy)-sulfonyl, -T-O-R¹⁰, wherein T is branched or linear C₁₋₈ alkanediyl and R¹⁰ is selected from the group consisting of hydrogen, methyl, substituted methyl, substituted ethyl, phenyl, substituted phenyl, substituted benzyl, pyridinylmethyl, substituted pyridinylmethyl, substituted silyl, C₁₋₆ acyl, substituted C₁₋₆ acyl, (C₁₋₄ alkoxy)carbonyl, substituted (C₁₋₄ alkoxy)carbonyl and aryloxycarbonyl; and
R⁹ may be located at any position of the phenyl ring.

7. The process of any of claims 1 to 6, wherein R¹ is of formula V, wherein R³ and R⁴ together form a moiety of -CR⁵=CR⁶-CR⁷=CR⁸-; R⁵, R⁶ and R⁸ are hydrogen; and R⁷ is chlorine; and
wherein R² is -C₆H₄R⁹, and R⁹ is methoxycarbonyl and located at position 2 of the phenyl ring.

8. The process of any of claims 1 to 7, wherein R¹ is of formula V, wherein R³ and R⁴ together form a moiety of -CR⁵=CR⁶-CR⁷=CR⁸-; R⁵, R⁶ and R⁸ are hydrogen; and R⁷ is chlorine; and
wherein R² is -C₆H₄R⁹, and R⁹ is -T-O-R¹⁰ wherein T is -C(CH₃)₂- and R¹⁰ is hydrogen or substituted methyl selected from the group consisting of tetrahydropyranyl, methoxymethyl and ethoxymethyl; and R⁹ is located at position 2 of the phenyl ring.

9. The process of any of claims 1 to 8, wherein each R¹¹ is phenyl; each R¹² is hydrogen; each Q is =CR¹⁵-, and wherein each R¹⁵ together with R¹³ bound to the same benzene ring and together with said benzene ring form a naphthalene ring system.

10. The process of any of claims 1 to 9, wherein the process is conducted in the presence of a base.

11. The process of any of claims 1 to 10, wherein the process is conducted in the presence of a Lewis acid.

12. The process of any of claims 1 to 11, wherein the process is conducted in the presence of a phase transfer catalyst.

13. The process of any of claims 1 to 12, wherein the process is conducted at a pressure between 1 and 100 bar.

14. The process of any of claims 1 to 13, wherein the process is conducted at an amount of the ketone of formula II (substrate) at a molar ratio relative to the catalyst (S/C) from 100:1 1 to 100,000:1.

15. The process of any of claims 1 to 14, wherein the process is conducted at a temperature between 0 °C and 100 °C.

16. The process of any of claims 1 to 15 for synthesizing an optically active alcohol of formula I as an intermediate in the preparation of 1-[[[1-[(*E*)-3-(2-(7-chloro-2-quinolinyl)ethenyl)phenyl]-3(*R*)-[2-(1-hydroxy-1-methylethyl)phenyl]propyl]-thio]methyl]cyclopropaneacetic acid (montelukast) or montelukast sodium.
